# EUROPEAN PATENT SPECIFICATION

(11) **EP 2 985 275 B1**
(45) Date of publication and mention of the grant of the patent: **30.10.2019**
(21) Application number: 13881969.3
(22) Date of filing: 06.12.2013
(51) Int. Cl.: C07C 51/42, C07C 59/01, C07C 51/29

(54) **BETA-HYDROXY-BETA-METHYLBUTYRIC ACID PURIFICATION METHOD**
VERFAHREN ZUR AUFREINIGUNG VON BETA-HYDROXY-BETA-METHYLBUTYLSÄURE
PROCÉDÉ DE PURIFICATION DE L'ACIDE BÉTA-HYDROXY-BÉTA-MÉTHYLBUTYRIQUE

(30) Priority: 12.04.2013 CN 201310127262
(43) Date of publication of application: 17.02.2016
(73) Proprietor: TSI (China) Co., Ltd., Shanghai 200233 (CN); TSI Pharmaceutical (Jiangyin) Co., Ltd., Jiangyin, Jiangsu 214446 (CN)
(72) Inventor: LONG, Ling, Shanghai 200233 (CN); TANG, Yongchun, Jiangyin Jiangsu 214446 (CN)
(74) Representative: Evans, Jacqueline Gail Victoria
(86) International application number: PCT/CN2013/088762
(87) International publication number: WO 2014/166273

(56) References cited:
- WO-A1-2013/025775
- CN-A- 1 417 190
- CN-A- 102 911 085
- US-A- 4 992 470
- US-A- 6 090 978
- US-A- 6 090 978
- MENG, JIN ET AL.: 'Optimal Control of Production Process for beta-Hydroxy-beta-Methyl Butyrate Calcium' HENAN CHEMICAL INDUSTRY 2002, pages 33 34 - 34, XP008180842 ISSN: 1003-3467

## Description

### Technical Field

The present invention belongs to the field of chemical synthesis. Specifically, the present invention relates to a method for purification of β-hydroxyl-β-methyl butyrate.

### Technical Background

β-Hydroxyl-β-methyl butyrate (HMB, C₅H₁₀O₃) is an intermediate metabolite of leucine, which is a kind of essential amino acid. Leucine is a branched amino acid and is essential. It is not produced *in vivo*, but it is very important to the human health. People have to rely on diet to ensure sufficient intake of leucine. HMB is an intermediate metabolite of leucine. HMB is contained in food, and the human body can also produce a little amount of HMB.

The currently marketed in the health product market is a monohydrate of the calcium salt of HMB, which generally is calcium β-Hydroxyl-β-methyl butyrate. Although its metabolic mechanism *in vivo* is unclear, it has been recommended for use against the degradation of muscle protein during resistive exercise. It can maximatily facilitate the increase of muscle volume. Investigators have proposed that HMB might be a component essential for muscle cell membrane under exercise stress, or it may regulate activity of some enzymes which are important for muscle growth. Animal tests on poultry, cattle and pig show that supplementing HMB may increase fat-free body weight and reduce body fat.

In the prior art, it is not difficult to synthesize HMB. However, it is somewhat difficult to obtain HMB of high purity. Currently, HMB is purified mainly by rectification or repetitious extraction and washing.

US 6090978A describes the isolation of calcium HMB by a method involving the steps of isolating crude HMB using a water stripping step, acidicfication using hydrochloric acid followed by extraction with ethyl acetate and addition of water and ethanol to the crude HMB and, addition of calcium hydroxide at a controlled rate. Following addition of celite, stirring of the resulting solution and filtering to remove the celite, seed crystals are added and calcium HMB is isolated.

US 4992470A describes a method for the isolation of crude HMB comprising acidifcation using sulphuric acid followed by extraction with ethyl acetate. The dried HMB acid is then neutalised with calcium hydroxide and dissolved in hot ethanol. Calcium HMB is crystallised from the ethanol solution and further purified by repeating the recrystallization step.

HMB is prone to produce impurities under high temperature, which influence its purity. Rectification cannot be carried out under normal pressure, but should be done under reduced pressure, 0,05 bar (40 mmHg) and about 120°C of boiling point. And the rectification cannot last too long. It has higher requirements on the devices, including being able to be operated under high temperature and high vacuum, and being resistant to corrosion by acid and chloride ion. As a result, none of the precision, structure and material of the device can satisfy the processing requirements. After rectification, there are still front cut fraction and cauldron bottom residue in addition to the product. If the front cut fraction and cauldron bottom residue are not be utilized, it might not be cost-effective. Therefore, problems, such as high requirement on device, high cost and easy production of by-product, are associated with the rectification method.

In the repetitious extraction and washing method, HMB is purified by multiple extractions and reverse extractions by utilizing different partition ratios of HMB and impurities in water and organic solvent under different pH values. However, it is difficult to obtain a product having purity up to 98% by this method. The purity obtained by this method is lower than those obtained by the rectification and the salification and crystallization method. Additionally, the product has a poor color.

Therefore, there is an urgent need in the art to develop an improved method for purifying HMB, which can simplify the process, reduce cost and improve the quality of the product.

### Summary of Invention

The present invention is intended to provide a method for purification of β-hydroxyl-β-methyl butyrate.

In the first aspect of the present invention, a method for purification of β-hydroxyl-β-methyl butyrate is provided, comprising:
(1) Neutralizing a crude β-hydroxyl-β-methyl butyrate with a base, crystallizing, centrifuging, and drying to obtain a crystallized product containing a salt of β-hydroxyl-β-methyl butyrate;
(2) adding the product containing a salt of β-hydroxyl-β-methyl butyrate obtained in step (1) into pure water in a weight ratio of 1:1-5, and dropping an inorganic acid at 0-40 °C, until the pH reaches 1-3.5 and extracting β-hydroxyl-β-methyl butyrate from the dissolved and acidified product to obtain β-hydroxyl-β-methyl butyrate of purity of greater than 98%.

In one preferred embodiment, in step (1), the neutralizing reaction comprises dissolving the crude β-hydroxyl-β-methyl butyrate; adding a base at 20-60 °C, preferably at 30-60 °C, until the pH reaches 6-8; adding diatomite and maintaining the temperature at 40-75 °C, preferably at 45-75 °C; filtering and recovering the filtrate; and slowly cooling to -10 to 20 °C to obtain the β-hydroxyl-β-methyl butyrate crystal.

In another preferred embodiment, the crude β-hydroxyl-β-methyl butyrate is dissolved in an aqueous solvent, or in ethanol.

In step (2), the dissolution and acidification comprises adding the product containing a salt of β-hydroxyl-β-methyl butyrate into pure water in a weight ratio of 1:1-5(salt of β-hydroxyl-β-methyl butyrate:water), preferably 1:1-3, dropping an inorganic acid at 0-40 °C, preferably 10-40 °C, until the pH reaches 1-3.5.

In an preferred embodiment, the base is a base, such as a divalent metal base, that can form a salt having low solubility with HMB, preferably the salt has a solubility of less than 50%, such as lower than 40%, lower than 30%, lower than 20%, lower than 10%. Preferably, the base is selected from, but is not limited to, calcium hydroxide and magnesium hydroxide (the salt of β-hydroxyl-β-methyl butyrate thus obtained is, for example, calcium β-hydroxyl-β-methyl butyrate, and magnesium β-hydroxyl-β-methyl butyrate).

In another preferred embodiment, the inorganic acid is selected from, but is not limited to, hydrochloric acid, sulfuric acid, nitric acid and phosphoric acid.

In another preferred embodiment, in step (2), an organic solvent is used to extract β-hydroxyl-β-methyl butyrate from the dissolved and acidified product to obtain an organic solvent extract, and then the organic solvent is removed to produce β-hydroxyl-β-methyl butyrate in a high purity.

In another preferred embodiment, the organic solvent is a water-immiscible solvent, and is selected from, but is not limited to, methyl acetate, ethyl acetate, butyl acetate, n-butanol, methyl ethyl ketone, methyl isobutyl ketone, dichloromethane, and dichloroethane.

In another preferred embodiment, the extraction includes extracting with the organic solvent for 2-5 times, with sufficiently stirring, standing and collecting the organic phase after each addition of the organic solvent.

In another preferred embodiment, in step (3), the organic solvent is removed by vacuum distillation.

In another preferred embodiment, the organic solvent is ethyl acetate, and the organic solvent is removed by vacuum distillation under the following conditions: a distillation temperature of ≤70 °C and a -0.09Mpa or above of vacuum degree in the posterior stage; and after the vacuum degree reaches -0.09Mpa or above, the temperature reaches 60-70 °C and no bubble produces, distillation is stopped.

In another preferred embodiment, before step (1), the method further comprises:
(a) reacting diacetone alcohol with hypochlorite to produce a product containing a salt of β-hydroxyl-β-methyl butyrate;
(b) reacting the product containing a salt of β-hydroxyl-β-methyl butyrate obtained in step (a) with an inorganic acid to obtain a crude β-hydroxyl-β-methyl butyrate.

Other aspects of the present invention will be apparent to the skilled artisan in view of the disclosed contents in the subject application.

### Specific Mode for Carrying Out the Invention

After repeated and thorough study, the inventors unexpectedly found an ideal method for purifying β-hydroxyl-β-methyl butyrate (HMB). The method comprises neutralizing a crude β-hydroxyl-β-methyl butyrate with a base to obtain a salt of β-hydroxyl-β-methyl butyrate, cooling and crystallizing, dissolving and acidifying, and then extracting β-hydroxyl-β-methyl butyrate in a high purity. No strict conditions, such as high temperature and high vacuum, are required in this method. On the contrary, it has low requirement on the apparatus, the process can readily be controlled and a product with high purity can be produced.

Currently, the crude β-hydroxyl-β-methyl butyrate is produced from the following chemical reactions. Firstly, diacetone alcohol is reacted with hypochlorite to produce a product containing Sodium β-hydroxyl-β-methyl butyrate; then the product containing Sodium β-hydroxyl-β-methyl butyrate is reacted with an acid to obtain an aqueous solution of the crude β-hydroxyl-β-methyl butyrate. The aqueous solution is extracted by organic solvent for several times and the organic phases are pooled. Then the solvents are removed under vacuum to produce the crude β-hydroxyl-β-methyl butyrate.

In the present invention, the crude β-hydroxyl-β-methyl butyrate is reacted with a base to produce a salt of β-hydroxyl-β-methyl butyrate. The product is cooled, crystallized, dissolved and acidified, and then β-hydroxyl-β-methyl butyrate of high purity is extracted therefrom with good purity and mild process conditions.

In the preferred embodiment of the present invention, the organic solvent is removed by distillation under reduced pressure. The distillation temperature is controlled to be not higher than 70 °C and the vacuum degree is controlled to be from low vacuum degree to high vacuum degree. As a result, loss of solvent can be decreased and the organic solvent, such as ethyl acetate, can be removed by distillation as much as possible.

No strict conditions, such as high temperature and high vacuum, are required in the method of the present invention. Thus, the present invention has a low requirement on the apparatus, the process can readily be controlled and a product with high purity can be produced. The qualities of the product of the present invention include: appearance, a colorless to light yellow, thick and transparent liquid; purity, higher than 98%.

Starting materials used in the present invention are commercial materials in the art. Preparation of the crude β-hydroxyl-β-methyl butyrate is not specifically limited in the present invention. Any known starting materials and operation conditions can be used in the present invention. It should be understood that no matter how the crude β-hydroxyl-β-methyl butyrate is obtained, as long as the purification is practiced according to the idea of the present invention after obtaining the crude β-hydroxyl-β-methyl butyrate, such process shall be contained within the protection scope of the present invention.

The present invention will be further illustrated be making reference to the following specific examples. It should be understood that these examples are only for illustrating the present invention but not for limiting the scope of the present invention. The experimental methods used in the following examples, the specific conditions of which are not specifically indicated, are carried out according to the conventional conditions, or according to the conditions recommended by the manufacturer. Unless specifically indicated, the percentage and the part are calculated based on weight.

Unless otherwise defined, all technical and scientific terms used herein have the same meaning as commonly known by the skilled artisan.

### Example 1: Production of HMB of high purity - production of crude HMB in the anterior stage

### 1. Starting materials and auxiliary materials

Starting materials and auxiliary materials used in the production of crude HMB in the early stage are shown in Table 1.

**Table 1**

| Name of the starting and auxiliary materials | Quality | Ratio |
|---|---|---|
| hypochlorite | Industrial grade | 1000kg |
| 4-Methyl-4-Hydroxyl-2-Pentanone (diacetone alcohol) | Industrial grade | Depending on the reaction status |
| Hydrochloric acid | Food grade | Depending on the pH |
| Ethyl acetate | Food grade | 1000kg |

### 2. Operational process and processing parameters

### 2.1 Oxidative synthesis (it is a strong exothermic reaction)

Sodium hypochlorite was pumped into a reaction kettle and diacetone alcohol (DIA) was slowly added. The addition velocity was controlled to maintain the reaction temperature of the materials in the kettle in the range of from 10 °C to 20 °C during the course of the reaction. DIA was stopped adding until the temperature significantly decreased when adding DIA, which indicated that reaction was no more taken place and came to an end.

### 2.2 Acidification

The reaction product obtained in 2.1 was pumped into a reaction kettle. The temperature was controlled to be about 20 °C. Hydrochloric acid was added to adjust the pH to 2∼3.5. After addition of the acid, the mixture was allowed to stand for 30 minutes. The by-product, chloroform, formed in the bottom was removed by observation by a sight glass.

### 2.3 Removal of water by evaporation

The reaction product obtained in 2.2 was pumped into a reaction kettle and subjected to vacuum concentration until the volume of the product was reduced to about half of its initial volume. The product was cooled to 60 °C or below, discharged from the kettle and filtered. The resultant sodium chloride was removed by filtration. The filtrates were pooled and added to the reaction kettle.

### 2.4 Extraction

The filtrate was extracted by ethyl acetate for three times. Before each extraction, the pH of the filtrate was adjusted to 2∼3.5 by hydrochloric acid. And after each addition of ethyl acetate, the resultant mixture was thoroughly stirred and then subjected to standing and layering. The ethyl acetate layer was collected.

### 2.5 Removal of ethyl acetate

The extracted ethyl acetate layers were pooled and pumped into a reaction kettle. Ethyl acetate was removed by vacuum distillation. The resultant product was cooled to 60 °C or below and discharged from the kettle to obtain a crude HMB .

### Example 2: Production of HMB of high purity - refining of HMB in the posterior stage

### 1. Starting auxiliary materials

Starting auxiliary materials used for refining the crude HMB are shown in Table 2.

**Table 2**

| Name of the starting and auxiliary materials | Quality | Addition amount (for each batch) |
|---|---|---|
| Crude HMB | Produced by the subject invention | 40 kg |
| Ethanol | Food grade | 200kg |
| Calcium hydroxide | Food grade | Depending on pH |
| Diatomite | Food grade | 2kg |
| Hydrochloric acid | Food grade | Depending on pH |
| Ethyl acetate | Food grade | 600 kg |

### 2. Operational process and processing parameters

### 2.1 Neutralization and crystallization

Ethanol was added into a reaction kettle. The crude HMB (about 40kg) prepared in Example 1 was added into the kettle, stirred and heated to 40∼60 °C. After the reaction was stable, calcium hydroxide was added to adjust the pH to 6.5∼7.5. Then 2kg Diatomite was added and the temperature was kept at 60∼65 °C. The reaction product was filtered to a crystallization kettle and slowly cooled to 0∼10 °C, and then kept at that temperature range.

### 2.2 Centrifugation

The reaction product obtained in 2.1 was added into a centrifuge having a laid filter cloth and centrifuged to obtain a wet product.

### 2.3 Drying

The wet product obtained after centrifugation was placed on a tray and put into an over for drying under 60∼80 °C. HMB-Ca was obtained after drying. This drying step could be omitted if lose of solvent is acceptable.

### 2.4 Dissolution and acidification

HMB-Ca obtained in 2.3 was weighed and pure water was added in a weight ratio of 3:1 (water: HMB-Ca). Hydrochloric acid was added under 20∼30□. The mixture was stirred and allowed HMB-Ca to dissolve. When pH reached 2-3, hydrochloric acid was stopped adding.

### 2.5 Extraction

The above dissolved and acidified solution was extracted by ethyl acetate for three times. For each addition of ethyl acetate, the resultant mixture was thoroughly stirred and subjected to standing and layering at 20∼30 °C. Then the ethyl acetate layers were collected.

### 2.6 Removal of solvent

The extracts obtained from the three extractions were pooled. Ethyl acetate was removed by vacuum distillation. The distillation temperature was controlled to 40∼70 °C and the vacuum degree in the posterior period was controlled to -0.09Mpa or above. When the vacuum degree reached -0.09Mpa or above, the temperature reached 65∼70 °C and no bubble was produced within the kettle, the distillation was stopped. The resultant product was cooled to 50 °C and discharged from the kettle to obtain HMB of high purity.

As tested, purity of the HMB obtained by the above method was 99.6% and the product has an excellent color.

### Example 3: Production of HMB of high purity - refining of HMB in the posterior stage

The starting and auxiliary materials, operational process and processing parameters used were the same as those used in Example 2, except that the neutralization and crystallization were carried out as follows:
The crude HMB (about 40kg) was pumped into a reaction kettle. Deionized water (120Kg) was added, and then calcium hydroxide was added under 30∼50 °C to adjust the pH to 6.5∼7.5. 2kg Diatomite was added and the temperature was raised to 65∼70□ and kept at this temperature range for 10 minutes. The reaction product was filtered to a reaction kettle and slowly cooled to 0∼10 °C.

Centrifugation, drying, dissolution and acidification, extraction and removal of solvent were identical to those of Example 2.

As tested, purity of the HMB obtained by the above method was 99.8% and the product has an excellent color.

### Example 4: Production of HMB of high purity - study on availability of starting and auxiliary materials

Production was performed with materials, operational process and processing parameters similar to those used in Example 2. Differences are listed in the following Table 3.

**Table 3**

| No. | Starting Materials | Process | Contents of HMB in the product |
|---|---|---|---|
| 1 | In step 2.1, calcium hydroxide was placed by magnesium hydroxide for neutralization | During neutralization, the pH was kept for 1.5 hours. Other processing conditions were the same as Example 2 | 99.2% |
| 2 | In Step 2.4, hydrochloric acid was replaced by sulfuric acid for acidification | Other processing conditions were the same as Example 2 | 99.5% |
| 3 | In Step 2.4, hydrochloric acid was replaced by nitric acid for acidification | Other processing conditions were the same as Example 2 | 98.8% |
| 4 | In Step 2.4, hydrochloric acid was replaced by phosphoric acid for acidification | Other processing conditions were the same as Example 2 | 98.7% |
| 5 | It step 2.5, ethyl acetate was replaced by methyl acetate for extraction | Other processing conditions were the same as Example 2 | 99.5% |
| 6 | It step 2.5, ethyl acetate was replaced by methyl ethyl ketone for extraction | Other processing conditions were the same as Example 2 | 99.2% |
| 7 | It step 2.5, ethyl acetate was replaced by methyl ethyl ketone for extraction | Other processing conditions were the same as Example 2, except that the posterior period of removal of solvent was maintained for 4 hours | 98.9% |
| 8 | It step 2.5, ethyl acetate was replaced by butyl acetate for extraction | Other processing conditions were the same as Example 2, except that the posterior period of removal of solvent was maintained for 4 hours | 99.3% |
| 9 | In step 2.5, n-butanol was used for extraction | Other processing conditions were the same as Example 2, except that the posterior period of removal of solvent was maintained for 4 hours | 99.4% |
| 10 | It step 2.5, ethyl acetate was replaced by iso-butanol for extraction | Other processing conditions were the same as Example 2, except that the posterior period of removal of solvent was maintained for 4 hours | 99.1% |
| 11 | In step 2.5, dichloromethane was used for extraction | Other processing conditions were the same as Example 2, except that the posterior period of removal of solvent was maintained for 4 hours | 98.4% |
| 12 | It step 2.5, ethyl acetate was replaced by dichloroethane for extraction | Other processing conditions were the same as Example 2, except that the posterior period of removal of solvent was maintained for 4 hours | 98.6% |

The contents of HMB in the final products were listed in Table 3. It can be found that products having high purity and excellent color could be obtained by suitable replacement of the starting materials.

## Claims

1. A method for purifying β-hydroxyl-β-methyl butyrate, comprising:
(1) Neutralizing a crude β-hydroxyl-β-methyl butyrate with a base, crystallizing, centrifuging, and drying to obtain a crystallized product containing a salt of β-hydroxyl-β-methyl butyrate;
(2) adding the product containing a salt of β-hydroxyl-β-methyl butyrate obtained in step (1) into pure water in a weight ratio of 1:1-5, and dropping an inorganic acid at 0-40 °C, until the pH reaches 1-3.5and extracting β-hydroxyl-β-methyl butyrate from the dissolved and acidified product to obtain β-hydroxyl-β-methyl butyrate of purity of greater than 98%.

2. The method of claim 1, wherein in step (1), the neutralizing and crystallizing stages comprise dissolving the crude β-hydroxyl-β-methyl butyrate; adding a base at 20-60 °C until the pH reaches 6-8; adding diatomite and maintaining the temperature at 40-75 °C; filtering and recovering the filtrate; and slowly cooling to -10 to 20 °C to obtain the β-hydroxyl-β-methyl butyrate crystal.

3. The method of claim 2, wherein the crude β-hydroxyl-β-methyl butyrate is dissolved in an aqueous solvent.

4. The method of claim 2, wherein the crude β-hydroxyl-β-methyl butyrate is dissolved in an ethanol solvent.

5. The method of any preceding claim, wherein the base is a metal salt having a solubility with β-hydroxyl-β-methyl butyrate of less than 50%.

6. The method according to claim 6, wherein the base is selected from calcium hydroxide and magnesium hydroxide.

7. The method of any preceding claim, wherein the inorganic acid is selected from the group consisting of hydrochloric acid, sulfuric acid, nitric acid and phosphoric acid.

8. The method of any preceding claim, wherein in step (2), an organic solvent is used to extract β-hydroxyl-β-methyl butyrate from the dissolved and acidified product to obtain an organic solvent extract, then the organic solvent is removed to produce β-hydroxyl-β-methyl butyrate of high purity.

9. The method of claim 8, wherein the organic solvent is a water-immiscible solvent, and is selected from the group consisting of methyl acetate, ethyl acetate, butyl acetate, n-butanol, methyl ethyl ketone, methyl isobutyl ketone, dichloromethane and dichloroethane.

10. The method of claim 8, wherein the organic solvent is removed by vacuum distillation.

11. The method of any preceding claim, wherein before step (1), the method further comprises:
(a) reacting diacetone alcohol with hypochlorite to produce a product containing a salt of β-hydroxyl-β-methyl butyrate;
(b) reacting the product containing a salt of β-hydroxyl-β-methyl butyrate obtained in step (a) with an inorganic acid to obtain a crude β-hydroxyl-β-methyl butyrate.

## Patentansprüche

1. Verfahren zum Aufreinigen von β-Hydroxyl-β-Methylbutyrat, umfassend:
(1) Neutralisieren eines rohen β-Hydroxyl-β-Methylbutyrats mit einer Base, Kristallisieren, Zentrifugieren und Trocknen, um ein kristallisiertes Produkt zu erhalten, das ein Salz von β-Hydroxyl-β-Methylbutyrat enthält;
(2) Zugeben des in Schritt (1) erhaltenen Produkts, das ein Salz von β-Hydroxyl-β-Methylbutyrat enthält, zu reinem Wasser in einem Gewichtsverhältnis von 1:1-5, und Tropfen einer anorganischen Säure bei 0 bis 40 °C, bis der pH-Wert 1 bis 3,5 erreicht, und Extrahieren von β-Hydroxyl-β-Methylbutyrat aus dem aufgelösten und saurem Produkt, um β-Hydroxyl-β-Methylbutyrat mit einer Reinheit von über 98% zu erhalten.

2. Verfahren nach Anspruch 1, wobei in Schritt (1) die Stufen des Neutralisierens und Kristallisierens das Auflösen des rohen β-Hydroxyl-β-Methylbutyrats umfasst; Zugeben einer Base bei 20 bis 60 °C, bis der pH-Wert 6 bis 8 erreicht; Zugeben von Diatomit und Aufrechterhalten der Temperatur auf 40 bis 75 °C; Filtern und Rückgewinnen des Filtrats; und langsames Abkühlen auf -10 bis 20°C, um β-Hydroxyl-β-Methylbutyrat-Kristall zu erhalten.

3. Verfahren nach Anspruch 2, wobei das rohe β-Hydroxyl-β-Methylbutyrat in einem wässrigen Lösungsmittel aufgelöst wird.

4. Verfahren nach Anspruch 2, wobei das rohe β-Hydroxyl-β-Methylbutyrat in einem Ethanol-Lösungsmittel aufgelöst wird.

5. Verfahren nach einem der vorstehenden Ansprüche, wobei die Base ein Metallsalz mit einer Löslichkeit mit β-Hydroxyl-β-Methylbutyrat von unter 50% ist.

6. Verfahren nach Anspruch 6, wobei die Base ausgewählt ist aus Calciumhydroxid und Magnesiumhydroxid.

7. Verfahren nach einem der vorstehenden Ansprüche, wobei die anorganische Säure ausgewählt ist aus der Gruppe bestehend aus Salzsäure, Schwefelsäure, Salpetersäure und Phosphorsäure.

8. Verfahren nach einem der vorstehenden Ansprüche, wobei in Schritt (2) ein anorganisches Lösungsmittel verwendet wird, um β-Hydroxyl-β-Methylbutyrat aus dem aufgelösten und sauren Produkt zu extrahieren, um ein anorganisches Lösungsmittelextrakt zu erhalten, wobei das anorganische Lösungsmittel danach entfernt wird, um β-Hydroxyl-β-Methylbutyrat mit hoher Reinheit zu erzeugen.

9. Verfahren nach Anspruch 8, wobei das anorganische Lösungsmittel ein mit Wasser nicht mischbares Lösungsmittel ist, und wobei es ausgewählt ist aus der Gruppe bestehend aus Methylacetat, Ethylacetat, Butylacetat, n-Butanol, Methylethylketon, Methylisobutylketon, Dichlormethan und Dichlorethan.

10. Verfahren nach Anspruch 8, wobei das anorganische Lösungsmittel durch Vakuumdestillation entfernt wird.

11. Verfahren nach einem der vorstehenden Ansprüche, wobei das Verfahren vor Schritt (1) ferner folgendes umfasst:
(a) Reagieren von Diacetonalkohol mit Hypochlorit, um ein Produkt zu erzeugen, das ein Salz von β-Hydroxyl-β-Methylbutyrat enthält;
(b) Reagieren des in Schritt (a) erhaltenen Produkts, das ein Salz von β-Hydroxyl-β-Methylbutyrat enthält, mit einer anorganischen säure, um ein rohes β-Hydroxyl-β-Methylbutyrat zu erhalten.

## Revendications

1. Procédé de purification de butyrate de β-hydroxyl-β-méthyl, comprenant les étapes consistant à :
(1) neutraliser un butyrate de β-hydroxyl-β-méthyle brut avec une base, cristalliser, centrifuger et sécher pour obtenir un produit cristallisé contenant un sel de butyrate β-hydroxyl-β-méthyle ;
(2) ajouter le produit contenant un sel de butyrate β-hydroxyl-β-méthyl obtenu à l'étape (1) dans de l'eau pure dans un rapport pondéral de 1:1-5, et verser en goutte à goutte un acide inorganique à 0-40 °C, jusqu'à ce que le pH atteigne 1-3,5 et extraire le butyrate β-hydroxyl-β-methyl du produit dissous et acidifié pour obtenir du butyrate β-hydroxyl-β-methyl ayant une pureté supérieure à 98 %.

2. Procédé selon la revendication 1, à l'étape (1), les étapes de neutralisation et de cristallisation comprenant les étapes consistant à dissoudre le butyrate de β-hydroxyl-β-méthyle brut ; ajouter une base à 20-60 °C jusqu'à ce que le pH atteigne 6-8 ; ajouter la diatomite et maintenir la température à 40-75 °C ; filtrer et récupérer le filtrat ; et refroidir lentement entre -10 et 20 °C pour obtenir le cristal de butyrate de β-hydroxyl-β-méthyle.

3. Procédé selon la revendication 2, le butyrate de β-hydroxyl-β-méthyl brut étant dissous dans un solvant aqueux.

4. Procédé selon la revendication 2, le butyrate de β-hydroxyl-β-méthyle brut étant dissous dans un solvant éthanol.

5. Procédé selon l'une quelconque des revendications précédentes, la base étant un sel métallique ayant une solubilité avec le butyrate de β-hydroxyl-β-méthyl inférieure à 50 %.

6. Procédé selon la revendication 6, la base étant choisie parmi l'hydroxyde de calcium et l'hydroxyde de magnésium.

7. Procédé selon l'une quelconque des revendications précédentes, l'acide inorganique étant choisi dans le groupe constitué par l'acide chlorhydrique, l'acide sulfurique, l'acide nitrique et l'acide phosphorique.

8. Procédé selon l'une quelconque des revendications précédentes, à l'étape (2), un solvant organique étant utilisé pour extraire le butyrate de β-hydroxyl-β-méthyl du produit dissous et acidifié pour obtenir un extrait de solvant organique, puis le solvant organique étant retiré pour produire du butyrate β-hydroxyl-β-méthyl de haute pureté.

9. Procédé selon la revendication 8, le solvant organique étant un solvant non miscible à l'eau et est choisi dans le groupe constitué par l'acétate de méthyle, l'acétate d'éthyle, l'acétate de butyle, le n-butanol, la méthyléthylcétone, la méthylisobutylcétone, le dichlorométhane et le dichloroéthane.

10. Procédé selon la revendication 8, le solvant organique étant retiré par distillation sous vide.

11. Procédé selon l'une quelconque des revendications précédentes, avant l'étape (1), le procédé comprenant en outre les étapes consistant à :
(a) faire réagir l'alcool diacétonique avec de l'hypochlorite pour produire un produit contenant un sel de butyrate de β-hydroxyl-β-methyl ;
(b) faire réagir le produit contenant un sel de butyrate de β-hydroxyl-β-méthyl obtenu à l'étape (a) avec un acide inorganique pour obtenir un butyrate de β-hydroxyl-β-methyl brut.
